# EUROPÄISCHE PATENTSCHRIFT

(11) **EP 0 526 397 B1**
(45) Veröffentlichungstag und Bekanntmachung des Hinweises auf die Patenterteilung: **17.01.1996**
(21) Anmeldenummer: 92810542.8
(22) Anmeldetag: 16.07.1992
(51) Int. Cl.: G01N 33/569

(54) **Immunologisches Nachweisverfahren**
Immunological detection method
Méthode de détection immunologique

(30) Priorität: 25.07.1991 CH 2237/91; 28.08.1991 CH 2517/91
(43) Veröffentlichungstag der Anmeldung: 03.02.1993
(73) Patentinhaber: CIBA-GEIGY AG, CH-4002 Basel (CH)
(72) Erfinder: Geiser, Martin, Dr., CH-4107 Ettingen (CH); Oddou Stock, Pascale, Dr., CH-4056 Basel (CH); Hartmann, Herbert, Dr., W-3004 Isernhagen 1 (DE)

(56) Entgegenhaltungen:
- JOURNAL OF CELL SCIENCE Bd. 83, 1986, LONDON UK Seiten 89 - 101 B.H. KNOWLES ET AL. 'Characterization and partial purification of a plasma membrane receptor for bacillus thuringiensis var. Kurstaki Lepidopteran-specific delta-endotoxin.'
- EUROPEAN JOURNAL OF BIOCHEMISTRY Bd. 186, Nr. 1-2, 1989, AMSTERDAM NL Seiten 239 - 247 J. VAN RIE ET AL. 'Specificity of Bacillus thuringiensis delta-endotoxin.'
- EUROPEAN JOURNAL OF BIOCHEMISTRY Bd. 202, Nr. 2, 1. Dezember 1991, AMSTERDAM NL Seiten 673 - 680 P. ODDOU ET AL. 'Identification and characterization of Heliothis virescens midgut membrane proteins binding Bacillus thuringiensis delta-endotoxin.'
- Experientia 38, 1982, pages 1103-1105

## Beschreibung

Die vorliegende Erfindung betrifft einen Antikörper, der spezifisch mit den Bindungsproteinen für *Bacillus thuringiensis* δ-Endotoxine reagiert. Sie betrifft weiterhin einen anti-idiotypischen Antikörper, der spezifisch mit den Bindungsproteinen *für B. thuringiensis* δ-Endotoxine reagiert. Ein weiterer Gegenstand vorliegender Erfindung ist ein Test-kit, der einen erfindungsgemässen Antikörper enthält. Die Erfindung betrifft ausserdem ein Verfahren zur Isolierung eines neuen *B. thuringiensis* δ-Endotoxins; ein Verfahren zur Identifizierung eines neuen *B. thuringiensis* δ-Endotoxins, das ein neues Bindungsprotein erkennt; ein Verfahren zur Bestimmung der Menge an vorhandenem Bindungsprotein und ein Verfahren zur Reduktion im Resistenzentwicklungspotential bei der Bekämpfung von Schädlingen. Ein weiterer Gegenstand vorliegender Erfindung ist die Verwendung von erfindungsgemässen Antikörpern und Test-kits in der Analyse der immunologischen Kreuzreaktivität von Toxin-Bindungsproteinen zur Bestimmung ihres Verwandschaftsgrads und zur Bestimmung der Verfügbarkeit und Zugänglichkeit eines Bindungsproteins für das entsprechende Toxin in verschiedenen Insekten. Ferner umfasst die vorliegende Erfindung die Verwendung der erfindungsgemässen Antikörper zur Analyse von Resistenzen, die auf das Fehlen des Bindungsproteins oder der Bindungsstellen zurückzuführen sind.

Während der Sporulation von *B. thuringiensis* werden kristalline Einschlusskörper produziert, welche Proteine enthalten, die für spezifische Insektenlarven letal sind. Diese Proteine sind als δ-Endotoxine oder als insektizide Kristallproteine (ICP) bekannt. Die verschiedenen ICPs können gemäss dem Schema von Höfte und Whiteley (1989) klassifiziert werden. Bekannt sind unter anderem die CryIA(a)-, CryIA(b)-, CryIA(c)- und CryIC-Toxine. Die nativen Kristallproteine sind inaktive Protoxine, nach der Aufnahme durch die Larven im alkalischen Insektendarm gelöst und proteolytisch aktiviert werden. Die aktivierten Toxine binden an ein oder mehrere Proteine an der Bürstensaummembran (BBM: Brush Border Membrane) der Mitteldarm-Epithelzellen von Zielinsekten und zerstören die Epithelzellen des Darrns, was zum Tod der Larven führt.

Im Rahmen der biologischen Schädlingsbekämpfung werden *B*. *thuringiensis* Toxine überwiegend in Form von Sporensuspensionen eingesetzt. Es sind aber auch verschiedene Pflanzen mit *B. thuringiensis* Toxingenen transformiert worden, um ihnen eine Resistenz gegenüber Schädlingen zu verleihen (beispielsweise EP 292,435, EP 317,511). Im Laufe der Zeit entwickelt sich bei den Schädlingen eine Resistenz gegenüber den beteiligten Toxinen, die mit der Bindungsfähigkeit von Toxin-Bindungsproteinen auf der BBM (Dixon, 1991; van Rie et al., 1990) verknüpft ist. Bestimmte Determinanten auf dem Toxin bestimmen die Spezifität der *B. thuringiensis* Toxine. Diese Determinanten sind in den variablen Regionen der C-terminalen Hälfte des aktivierten Toxins lokalisiert (Caramori et al., 1991; Ge et al., 1989; Schnepf et al., 1990).

Geht das Bindungsprotein für ein bestimmtes Toxin verloren oder ist seine Erkennungsregion so verändert, dass es das bestimmte Toxin nicht mehr bindet, so geht die Empfindlichkeit des Insekts gegenüber dem betreffenden Toxin verloren, es ist resistent. Durch den Einsatz verschiedener Toxine, die an unterschiedliche Bindungsproteine binden, kann einer raschen Resistenzentwicklung vorgebeugt werden. Auf Grund der Kenntnis des Zusammenspiels von jeweiligem Toxin und entsprechendem Bindungsprotein ist es möglich, solche Toxine auszuwählen und zu kombinieren, die verschiedenartige Bindungsproteine in der BBM des Mitteldarmepithels von Zielinsekten besetzen (EP 400,246).*B. thuringiensis* und Pflanzen können mit den entsprechenden Genen der so kombinierten Toxine transformiert werden. Die Wahrscheinlichkeit, dass Insekten gegen die rekombinanten *B.* *thuringiensis* oder die transgene Pflanze resistent werden, ist dadurch signifikant verringert, denn um gleichzeitig gegen Toxine resistent zu werden, die an verschiedenartige Bindungsproteine binden, sind mehrere gleichzeitige Mutationen erforderlich (EP 400,246).

Das Zusammenspiel von Toxin und Bindungsprotein kann unter anderem mit Hilfe immunologischer Verfahren untersucht werden. Derartige Verfahren werden bereits seit geraumer Zeit zur Analyse verwendet Es wird dabei die spezifische Bindung zwischen Antigen und Antikörper ausgenutzt. Das Antigen, d. h. die zu analysierende Verbindung, wird einem Säuger, beispielsweise einem Kaninchen, einer Maus oder einer Ratte, mehrmals in mehrwöchigem Abstand injiziert. Das Immunsystem des Tieres produziert daraufhin Antikörper, die sich spezifisch an das Antigen binden und deren Bindungsregion ein negatives Abbild der Bindungsregion des Antigens darstellt.

Durch die Verschmelzung der Antikörper-produzierenden Zellen mit beispielsweise Myelomazellen werden sie zu sogenannten Hybridomazellen, mit deren Hilfe monoklonale Antikörper hergestellt werden können. Die dabei angewendeten Verfahren sind in der Literatur beschrieben und einer Person, die fachlich qualifiziert ist, bekannt

Die vorliegende Erfindung betrifft einen Antikörper, der spezifisch mit den Bindungsproteinen für *B. thuringiensis* δ-Endotoxine und deren Abkömmlinge reagiert. Eine bevorzugte Ausführungsform ist ein Antikörper, der spezifisch mit den Bindungsproteinen für das *B*. *thuringiensis* CryIA(b) δ-Endotoxin und dessen Abkömmlinge reagiert, vorzugsweise ein Antikörper, der spezifisch mit dem *Heliothis* Bindungsprotein für das *B. thuringiensis* ... CryIA(b) δ-Endotoxin und dessen Abkömmlinge reagiert, insbesondere ein Antikörper, der spezifisch mit dem *H. virescens* 170 kDa Bindungsprotein für das *B. thuringiensis* CryIA(b) δ-Endotoxin und dessen Abkömmlinge reagiert.

Unter Abkömmlingen von *B. thuringiensis* δ-Endotoxinen sind im Rahmen der vorliegenden Erfindung insektizide Derivate dieser Toxine zu verstehen, die durch Modifikationen, chemischer oder mikrobiologischer Natur, erhältlich sind.

Der erfindungsgemässe Antikörper ist erhältlich, indem Bindungsproteine für *B. thuringiensis* δ-Endotoxine und deren Abkömmlinge aus BBM Vesikeln des Mitteldarms von Insekten gewonnen und analog bekannter Verfahren (siehe beispielsweise Harlow und Lane, 1988) zur Erzeugung der besagten Antikörper eingesetzt werden. Es kann beispielsweise so vorgegangen werden, dass (a) Bindungsproteine für *B. thuringiensis* δ-Endotoxine und deren Abkömmlinge aus BBM Vesikeln des Mitteldarms von Insekten gewonnen werden, (b) ein Säuger, beispielsweise ein Kaninchen, eine Maus oder eine Ratte mit den Bindungsproteinen immunisiert wird und (cl) die Immunglobuline selektioniert werden, die gegen die Bindungsproteine für *B. thuringiensis* δ-Endotoxine und deren Abkömmlinge gerichtet sind, oder (c2) Milzzellen des immunisierten Tieres mit entsprechenden Myelomazellen fusioniert, bestimmte Hybridomazellen selektioniert und unter Verwendung dieser Hybridomazellen die gewünschten Antikörper produziert werden.

Die Herstellung von BBM Vesikeln aus dem Mitteldarm von Insekten ist bekannt. Beispielsweise kann folgendermassen verfahren werden: Larven im 4. Larvenstadium werden 15 min auf Eis gekühlt Vorsichtig werden die Mitteldärme aus den Larven entnommen. Jeder Mitteldarm wird mit einem longitudinalen Schnitt geöffnet und der Darminhalt mit Hoyles Ringerlösung (140 mM NaCl: 9,4 mM KCl; 3,95 mM MgCl₂· 6 H₂O; 3,6 mM NaHCO₃; 6,55 mM Na₂HPO₄; 5,44 mM CaCl₂, pH 7,2) herausgespült. Nach dem Entfernen der peritrophen Membran werden die Mitteldärme sofort in 300 mM Mannit; 5 mM EGTA; 17 mM Tris-HCl pH 7,5 eingefroren und bis zum Gebrauch bei -70°C aufbewahrt.

Anschliessend werden BBM Vesikel (BBMV) von *H. virescens*, *H. zea, Spodoptera* *littoralis, S. exigua* und *S. litura* gemäss Wolfersberger et al. (1987) unter Verwendung der folgenden Protease-Inhibitoren hergestellt: 1 µg/ml Leupeptin, 1 µg/ml Antipain, 5 µg/ml Aprotinin, 10 g/ml Trypsin-Inhibitor aus Soja, 10 µg/ml Benzamidinhydrochlorid, 1 µg/ml Pepstatin A, 1 mM PMSF. Das Pellet, das am Schluss erhalten wird, wird in PBS resuspendiert oder in einem Puffer gelöst, der 40 mM Tris-HCl pH 7,5, 10 mM MgCl₂, 5 mM EGTA, 30 % Glycerin und 10 mM CHAPS enthält. Der Proteingehalt der BBMV wird mit BSA als Standard nach dem Verfahren von Bradford (1976) bestimmt. SDS-PAGE wird gemäss Laemmli (1970) durchgeführt.

Das CryIA(b)-Bindungsprotein aus *H. virescens* wird beispielsweise über eine präparative SDS-PAGE im Puffersystem von Laemmli (1970) gewonnen. Nach der Elektrophorese wird eine Spur mit Coomassie Blue-Lösung gefärbt, um das Bindungsprotein zu lokalisieren. Die Bande, die das 170 kDa Bindungsprotein enthält, wird aus dem Gel herausgeschnitten und in kleine Stücke zerteilt. Die Stücke werden anschliessend durch ein feines Metallnetz gepresst, um eine zur Injektion geeignete Suspension herzustellen.

Ein weiterer Gegenstand der vorliegenden Erfindung ist ein anti-idiotypischer Antikörper, der spezifisch mit den Bindungsproteinen für *B*. *thuringiensis* δ-Endotoxine und deren Abkömmlinge reagiert. Eine bevorzugte Ausführungsform ist ein anti-idiotypischer Antikörper, der spezifisch mit den Bindungsproteinen für das *B. thuringiensis* CryIA(b) δ-Endotoxin und dessen Abkömmlinge, vorzugsweise mit den *Heliothis* Bindungsproteinen, insbesondere mit dem *H*. *virescens* 170 kDa Bindungsprotein für das *B. thuringiensis* CryIA(b) δ-Endotoxin und dessen Abkömmlinge reagiert.

Anti-idiotypische Antikörper werden vom Immunsystem eines Tieres produziert, wenn die bereits gegen das eigentliche Antigen gewonnenen Antikörper nun selbst als Antigen zur Immunisierung des Tieres verwendet werden. Die Bindungsregion der anti-idiotypischen Antikörper ist ein Abbild der Bindungsregion des ursprünglichen Antigens.

Besonders bevorzugt im Rahmen der vorliegenden Erfindung ist ein anti-idiotypischer Antikörper, der spezifisch mit bestimmten Regionen der Bindungsproteine reagiert.

Ein erfindungsgemässer anti-idiotypischer Antikörper ist erhältlich, indem Antikörper gegen das native aktivierte Toxin gewonnen und analog bekannter Verfahren zur Erzeugung der anti-idiotypischen Antikörper eingesetzt werden. Es kann beispielsweise so vorgegangen werden, dass Antikörper gegen das native aktivierte Toxin erzeugt werden und mit diesen Antikörpern erneut immunisiert wird. Anschliessend werden beispielsweise aus dem Serum der immunisierten Tiere die gegen die Bindungsproteine für *B*. *thuringiensis* δ-Endotoxine und deren Abkömmlinge gerichteten Immunglobuline selektioniert oder es werden Milzzellen der immunisierten Tiere mit entsprechenden Myelomazellen fusioniert, bestimmte Hybridomazellen selektioniert und unter Verwendung dieser Hybridomazellen der gewünschte anti-idiotypische Antikörper produziert

Native aktivierte Toxine werden beispielsweise gewonnen, indem folgendermassen verfahren wird: *B. thuringiensis* HD1cryB wird jeweils mit einem der Plasmide pXI93, pXI94 und pXI95 gemäss der in EP 342,633 für pXI93 beschriebenen Methode transformiert und anschliessend kultiviert. Nach der Zell-Lyse werden übriggebliebene nicht-lysierte Zellen mit einem Zell-Beschallungsgerät zerstört und Sporen-KristallMischungen geerntet. Die Kristalle werden gemäss Delafield et al. (1968) gelöst und durch eine dreistündige Inkubation bei 28°C in einem Puffer (8 g/l NaCl, 0,2 g/l KCl, 0,05 g/l NaH₂PO₄ · 2 H₂O, 1 g/l Glucose, 1 g/l NaHCO₃, 0,68 g/l Na-Citrat pH 6,55) aktiviert, der 0,05 % Trypsin und 10 mM Triethanolamin pH 10,2 enthält. Im Fall von CryIC-Toxin wird DTT bis zu einer Endkonzentration von 5 mM zum Puffer hinzugefügt. Die restlichen Sporen oder nichtgelöste Kristalle werden durch Zentrifugation, 10.000 x g, 10 min, 4°C, entfernt.

Antikörper gegen das native aktivierte CryIA(b)-Toxin werden erzeugt, indem dem oben beschriebenen Immunisierungsplan unter Verwendung von 30 µg CryIA(b)-Toxin gefolgt wird. Booster-Injektionen werden vier Wochen, drei Monate und vier Monate später verabreicht. Die Applikationen erfolgen subcutan. Blutproben werden 12 Tage nach einer Immunisierung und später alle zwei Wochen genommen. Die IgGs werden über eine Säule mit dem Elutionsmittel 0,1 M Essigsäure und 0,15 M NaCl aufgetrennt, die Protein A, gekoppelt an vernetzte Agarose enthält.

Ein anti-idiotypischer Antikörper, der spezifisch mit bestimmten Regionen der Bindungsproteine für *B. thuringiensis* δ-Endotoxine und deren Abkömmlinge reagiert, ist erhältlich, indem (a) Antikörper gegen das native aktivierte *B. thuringiensis* δ-Endotoxin gewonnen werden, (b) von diesen Antikörpern diejenigen Antikörper, die nicht gegen bestimmte Regionen des Toxins gerichtet sind, durch subtraktive Affinitätschromatographie abgetrennt werden, (c) mit diesen Antikörpern, die gegen die bestimmte Region des Toxins gerichtet sind, erneut immunisiert wird, und (d1) die Immunglobuline selektioniert werden, die gegen bestimmte Regionen der Bindungsproteine für *B. thuringiensis* δ-Endotoxine und deren Abkömmlinge gerichtet sind, oder (d2) Milzzellen des immunisierten Tieres mit entsprechenden Myelomazellen fusioniert, bestimmte Hybridomazellen selektioniert und unter Verwendung dieser Hybridomazellen die gewünschten anti-idiotypischen Antikörper produziert werden.

Bei einer subtraktiven Affinitätschromatographie werden Antikörper, die spezifisch mit einem bestimmten Toxin reagieren und ausschliesslich Determinanten dieses Toxins erkennen, von solchen Antikörpern abgetrennt, die mit einem anderen Toxin oder mehreren anderen Toxinen kreuzreagieren. Dazu werden Antikörper gegen dieses bestimmte, native aktivierte Toxin über eine Säule gegeben, die mit einer Matrix gefüllt ist, an die dieses Toxin gekoppelt ist. Das Matrix-gebundene Toxin bindet ein Gemisch von Antikörpern, die gegen das gesamte Toxin gerichtet sind. Ein Teil dieser Antikörper kreuzreagiert mit anderen Toxinen. Um diese Antikörper abzutrennen, wird das Antikörpergemisch von der Säule eluiert, beispielsweise mit einer Lösung von Diethylamin und Desoxycholat, und nachfolgend über eine zweite Säule gegeben, die mit einer Matrix gefüllt ist, an die ein zweites Toxin gekoppelt ist oder an die mehrere unterschiedliche Toxine gekoppelt sind. Das Toxin oder die Mischung der unterschiedlichen Toxine ist so gewählt, dass alle Antikörper, die eine bestimmte Region des bestimmten Toxins nicht erkennen, gebunden werden und diejenigen Antikörper, die spezifisch an eine bestimmte Region des bestimmten Toxins binden, im Durchfluss zu finden sind. Als Matrix kann dabei beispielsweise vernetzte Agarose dienen, an die Protein A gekoppelt ist. Zweckdienliche Materialien sind in der Literatur beschrieben.

Ein wesentlicher Aspekt der vorliegenden Erfindung ist der Nachweis von Bindungen von Antikörpern an Toxine, von Toxinen an Bindungsproteine und von Antikörpern an Bindungsproteine, der gemäss üblichen, dem Fachmann bekannten Verfahren autoradiographisch oder immunologisch (beispielsweise gemäss Harlow und Lane, 1988) erfolgt.

Die Bindung der Antikörper an das CryIA(b) BP von *H. virescens* kann beispielsweise folgendermassen bestimmt werden:
(a) BBM Proteine werden durch SDS-PAGE aufgetrennt und durch Elektrotransfer (0,4 A; 1 h) auf eine Membran übertragen. Die Absättigung unspezifischer Bindestellen erfolgt durch Inkubation in TBSTM (1h). Die Membran wird anschliessend mit einer geeigneten Verdünnung der Antikörper in TBSTM mindestens 2 h lang inkubiert Danach werden die ungebundenen Antikörper durch Waschen mit TBST entfernt. Es folgt eine Inkubation der Membran mit Ziege-gegen-Maus-Antikörpern, die mit alkalischer Phosphatase markiert sind Ungebundene Antikörper werden durch Waschen mit TBST entfernt. Die gebundenen Antikörper werden durch Umsetzung mit NBT und BCIP in 0,1 M NaHCO₃, 1 mM MgCl₂, pH 9,8 sichtbar gemacht.
(b) BBM-Proteine werden mit Hilfe einer sogenannten Slot-Blot-Apparatur (Schleicher und Schüll) auf eine Membran transferiert. Nach Absättigung in TBSTM wird die Membran mit einer geeigneten Verdünnung der Antikörper in TBSTM inkubiert. Nachdem ungebundene Antikörper durch Waschen entfemt worden sind, wird die Membran mit einem gegen diesen Antikörper gerichteten, ¹⁵I markierten Antikörper inkubiert. Anschliessend werden ungebundene Antikörper durch Waschen entfernt und die Intensität der autoradiographischen Signale gemessen.

Ein Gegenstand der vorliegenden Erfindung ist ein Test-kit, der auf einem erfindungsgemässen Antikörper basiert.

Eine spezifische Ausführungsform dieser Erfindung ist ein Test-Kit, der auf einem der üblicherweise verwendeten Immunassays basiert, beispielsweise ausgewählt aus der Gruppe bestehend aus Radioimmunassay, Enzym-gekoppelter Immunassay und Chemilumineszenzassay. Die Rezepturen für derartige Test-kits sind von der jeweils gewählten Nachweismethode abhängig und sind dem Fachmann geläufig.

Ein weiterer Gegenstand der vorliegenden Erfindung betrifft ein Verfahren zur Isolierung eines neuen *B. thuringiensis* δ-Endotoxins, indem (a) die Bindungsproteine für ein vorgegebenes δ-Endotoxin durch die Bindung von Antikörpern abgesättigt werden und (b) das neue Toxin an andere Bindungsproteine von vorbehandelten Mitteldarm-Membran-Proteinen bindet.

Ein weiteres Verfahren zur Isolierung eines neuen *B. thuringienris* δ-Endotoxins, beruht darauf, daß spezifische Antikörper, die durch subtraktive Affinitätschromatographie gegen bekannte Toxine gewonnen werden, an eine Matrix gebunden werden und diese Matrix mit der Gesamtheit der Toxine eines Stammes oder mehrerer Stämme inkubiert wird, wobei ein neues Toxin mit anderen Strukturmerkmalen von den Antikörpern nicht erkannt und deshalb nicht gebunden wird und in der Lösung verbleibt.

Unter einem "neuen" *B. thuringiensis* δ-Endotoxin versteht man im Zusammenhang mit dieser Erfindung ein bisher noch nicht identifiziertes oder identifizierbares δ-Endotoxin.

Wieder ein anderen Verfahren zur Idendifizierung eines neuen *B*. *thuringiensis* δ-Endotoxins, das ein neues Bindungsprotein erkennt beruht darauf, daß die Bindungsproteine der BBMV verschiedener Insekten für bekannte δ-Endotoxine durch die Bindung mit diesen δ-Endotoxinen abgesättigt werden und geprüft wird, ob das neue Toxin dennoch bindet.

Ein weiterer Gesichtspunkt ist ein Verfahren zur Bereitstellung eines Gens, das ein neues Toxin kodiert, indem aus der Aminosäuresequenz des neuen Toxins die Nukleotidsequenz ganz oder teilweise abgeleitet wird und analog bekannter Verfahren entweder ein entsprechendes Gen synthetisiert oder mit Hilfe einer Sonde das natürlich vorkommende Gen identifiziert und anschliessend isoliert wird. Ebenfalls umfasst ist ein Verfahren zur Bereitstellung des Gens für ein neues Toxin, indem eine Genbank des Spenderbakteriums zur Expression gebracht wird, mit einem Antikörper gegen das neue Toxin die Expressionsprodukte geprüft werden und aus einem positiven Klon das gesuchte Gen gemäss allgemein üblichen, dem Fachmann geläufigen Verfahren isoliert wird. Selbstverständlich können alle anderen bekannten Verfahren zur Isolierung des Gens angewendet werden.

Die erfindungsgemässen Antikörper erlauben eine Analyse von Resistenzen. So ist es möglich, zu prüfen, ob ein bestimmtes Bindungsprotein vorhanden ist. Darüber hinaus ist es möglich, unter Verwendung eines erfindungsgemässen anti-idiotypischen Antikörpers zu bestimmen, ob bei vorhandenen Bindungsproteinen die Bindungsstellen verändert sind. Die vorliegende Erfindung betrifft somit die Verwendung eines erfindungsgemässen Antikörpers zur Analyse von Resistenzen, die auf das Fehlen des Bindungsproteins oder das Fehlen der Bindungsstellen zurückzuführen sind, sowie ein Verfahren zur Reduktion im Resistenzentwicklungspotential bei der Bekämpfung von Schädlingen, indem Veränderungen in der Anzahl der Bindungsstellen bzw. der Bindungsproteine bestimmt werden und dementsprechend ein geeignetes Toxin oder Toxingemisch eingesetzt wird.

Ein weiterer Gegenstand der vorliegenden Erfindung ist ein Verfahren zur Bestimmung der Menge an vorhandenem Bindungsprotein mit Hilfe eines erfindungsgemässen Antikörpers gegen dieses Bindungsprotein oder eines Test-kits, der einen erfindungsgemässen Antikörper enthält. Das Verfahren besteht im wesentlichen darin, dass die Probe, die die Bindungsproteine enthält, mit den Antikörpern oder dem Test-kit inkubiert wird und anschliessend die Menge an Bindungsprotein-Antikörper-Komplexen bestimmt wird.

Mit den erfindungsgemässen Antikörpern und Test-kits kann der Verwandschaftsgrad von Toxin-Bindungsproteinen bestimmt werden, indem die immunologische Kreuzreaktivität der Bindungproteine bestimmt wird. Die vorliegende Erfindung betrifft somit die Verwendung eines erfindungsgemässen Antikörpers oder Test-kits in der Analyse der immunologischen Kreuzreaktivität von Toxin-Bindungsproteinen zur Bestimmung ihres Verwandschaftsgrades, indem die Bindung eines Antikörpers gegen ein bestimmtes Bindungsprotein an immobilisierte Bindungsproteine verschiedener Herkunft bestimmt wird.

Die erfindungsgemässen Antikörper und Test-kits ermöglichen es, verschiedene Insekten auf die Verfügbarkeit und Zugänglichkeit eines Bindungsproteins für das entsprechende Toxin hin zu untersuchen. Mit diesen Antikörpern und Test-kits ist eine einfache und schnelle Identifizierung des Bindungsproteins in der Darmmembran von Zielinsekten möglich. Die vorliegende Erfindung betrifft somit die Verwendung eines erfindungsgemässen Antikörpers oder Test-kits zur Bestimmung der Verfügbarkeit und Zugänglichkeit eines Bindungsproteins für das entsprechende Toxin in verschiedenen Insekten.

### Abkürzungen

- BBM(V): Brush border membrane (vesicle) = Bürstensaummembran(vesikel)
- BCIP: 5-Brom-4-chlor-3-indolylphosphattoluidin Salz
- BP: Bindungsprotein
- BSA: Bovine serum albumin = Rinderserumalbumin
- CHAPS: 3-[(3-Cholamidpropyl)dimethylammonio]-1-propansulfonat
- DTT: Dithiothreitol
- EDAC: 1 -Ethyl-3-(3-dimethylaminopropyl)carbodiimid
- EGTA: [Ethylenbis(oxyethylennitrilo)]tetraessigsäure
- ICP: Insecticidal Crystal Protein = insektizides Kristallprotein
- IgG: Immunglobulin G
- NBT: p-Nitro Blau Tetrazoliumchlorid
- PBS: Phosphate buffered saline = Phosphat gepufferte Kochsalzlösung
- PMSF: Phenylmethyl-sulfonylfluorid.
- PVDF: Polyvinyliden-difluorid
- SDS-PAGE: Sodium dodecylsulfate polyacrylamide gel electrophoresis = Natriumdodecylsulfat-Polyacrylamidgelelektrophorese
- TBSTM: TBST (10 mM Tris-HCl pH 8,0, 150 mM NaCI und 0,5 % Polyoxyethylen-Sorbit-monolaurat) + 1 % fettfreie Trockenmilch

### Beispiele

### Bakterienstämme und Plasmid-DNA

*B. thuringiensis* HD1cryB ist als *B. thuringiensis* HD1cryβ gemäss Budapester Vertrag hinterlegt (DSM 4574).
pXI93 ist als pK93 gemäss Budapester Vertrag hinterlegt (DSM 4571)[enthält das cryIA(b)-Gen aus *B. thuringiensis kurstaki* HD1 (DSM 3667)].

### Hinterlegungen

Im Zusammenhang mit der vorliegenden Erfindung wurden die folgenden Plasmide bei der Deutschen Sammlung von Mikroorganismen gemäss Budapester Vertrag hinterlegt:

| DSM-Nummer | Datum | Plasmid |
|---|---|---|
| DSM 6616 | 23.7.1991 | pXI94[enthält das cryIA(a)-Gen aus *B. thuringiensis kurstaki* HD1] |
| DSM 6615 | 23.7.1991 | pXI95[enthält das cryIA(c)-Gen aus *B. thuringiensis kurstaki* HD73] |
| DSM 6614 | 23.7.1991 | pXI109[enthält das cryIC-Gen aus *B. thuringiensis* GC91 (NCTC 11821)] |

### Beispiel 1: CryIA(a)-, CryIA(b)-, CryIA(c)- und CryIC-Toxine und ihre Bindung an BBM-Proteine von Heliothis und Spodoptera

Nach SDS-PAGE (Gesamtprotein 10 µg) werden die denaturierten BBM-Proteine aus den Mitteldärmen von *H. virescens, H. zea, S. littoralis, S. exigua* und S. *litura* Larven 1 h lang bei 4°C mit 0,4 A auf eine PVDF-Membran übertragen (Towbin et al., 1979). Die Membran wird mit Ponceau S (0,2 % in 3 % Trichloressigsäure) (Serva) gefärbt, um die Proteine sichtbar zu machen. Unspezifische Bindungen werden durch dreissigminütige Inkubation bei Raumtemperatur in TBSTM verhindert. Die Membran wird über Nacht mit je 1,5 µg/ml aktivierten CryIA(a)-, CryIA(b)-, CryIA(c)- und CryIC-Toxinen inkubiert und das ungebundene Toxin durch Waschen mit TBST entfernt. Gebundenes Toxin wird mit dem monoklonalen Antikörper 82.1 (Huber-Lukac et al., 1986) oder mit Kaninchen-Antikörpern, die gegen CryIC (Beispiel 3) gerichtet sind (Serum 1:1000 in TBSTM verdünnt), identifiziert. Die ungebundenen Antikörper werden durch Waschen mit TBST entfernt. Anschliessend folgt eine einstündige Inkubation mit 1:5000 verdünnten, mit alkalischer Phosphatase markierten Ziegen-gegen-Maus-Antikörpern oder Ziegen-gegenKaninchen-Antikörper bei Raumtemperatur. Durch Umsetzung mit NBT und BCIP in 0,1 M NaHCO₃ und 1 mM MgCl₂, pH 9,8 wird der Membran-gebundene Komplex sichtbar gemacht.

Die drei CryIA-Toxine und das CryIC-Toxin erkennen ein oder mehrere Bindungsproteine im Mitteldarm jeder Insektenart (Tabelle 1).

**Tabelle 1:**

| *B. thuringiensis* Toxin-Bindungsproteine im Mitteldarm verschiedener Insekten (Molekulargewichte, kDa; SDS-PAGE) | | | | | |
|---|---|---|---|---|---|
| | *H. zea* | *H. virescens* | *S. littoralis* | *S. exigua* | *S. litura* |
| CryIA(a) | 170 | 170 | 160 | 200 | 150 |
| | | | | 180 | |
| CryIA(b) | 170 | 170 | 160 | 200 | 150 |
| | | | | 180 | |
| CryIA(c) | 150 | 140 | 125 | 130 | 125 |
| | 140 | 120 | 115 | 115 | |
| | 120 | | | | |
| CryIC | nd | 40 | 40 | 40 | 40 |

In allen Fällen erkennen CryIA(a) und CryIA(b) dieselben Proteine, von einer Insektenart zur anderen sind die Bindungsproteine jedoch unterschiedlich. CryIA(c) bindet an eine Reihe verschiedener Toxin-Bindungsproteine. CryIC bindet bei allen drei *Spodoptera-Arten* und bei *H*. *virescens* an ein Bindungsprotein mit einem Molekulargewicht von 40 kDa.

### Beispiel 2: Antikörper gegen das CryIA(b) BP von H. virescens

Ein Aliquot einer Suspension, das 15 µg CryIA(b)-Bindungsprotein aus *H*. *virescens* im Gel fixiert enthält, wird subcutan in den Rücken eines Chinchilla-Kaninchens injiziert. Booster-Injektionen von 15 µg Protein werden nach 3 und 7 Wochen verabreicht. Zwei Wochen nach der letzten Booster-Injektion wird Serum gewonnen. Das Serum wird vom Vollblut abgetrennt, indem man es gerinnen lässt und niedertourig abzentrifugiert. Das Serum wird in kleinen Aliquots bei -20°C bis zum Gebrauch aufbewahrt.

Um den Hintergrund zu reduzieren, den man erhält, wenn dieses Antiserum in Western Blots verwendet wird, werden unspezifische Antikörper mit einem an eine Affinitätschromatographie-Säule gekoppelten *E*. *coli-Lysat* (Sambrook et al., 1989) entfernt.

**Immunologische Unterschiede zwischen den CryIA(b) BP** Die Antikörper gegen das CryIA(b) BP von *H*. *virescens* werden für eine Immunblotting-Analyse von BBM Proteinen der Larven von fünf verschiedenen Insektenarten verwendet. Dazu werden je 3 µg BBM-Proteine mit Hilfe einer sogenannten Slot-Blot-Apparatur (Schleicher und Schüll) auf eine Membran transferiert. Nach Absättigung in TBSTM wird die Membran mit einer geeigneten Verdünnung der Antikörper in TBSTM inkubiert. Nachdem ungebundene Antikörper durch Waschen entfernt worden sind, wird die Membran mit ¹⁵I markierten Ziegen gegen Kaninchen-Antikörpern inkubiert. Anschliessend werden ungebundene Antikörper durch Waschen entfernt und die Intensität der autoradiographischen Signale mit einem Shimadzu CS-930 TLC Scanner gemessen.

Die Antikörper kreuzreagieren nur mit dem Bindungsprotein von *H*. *zea* aber nicht mit den Proteinen der *Spodoptera-*Arten. Obwohl das CryIA(b)-Toxin ein Bindungsprotein bzw. mehrere Bindungsproteine in jedem Insekt erkennt, ist das Bindungsprotein von *Heliothis* immunologisch nicht mit den Bindungsproteinen von *Spodoptera* verwandt.

### Beispiel 3: Antikörper gegen das CryIC-Toxin

Antikörper gegen das aktivierte CryIC-Toxin werden nach einem Standardverfahren erhalten: 10 µg aktiviertes CryIC-Toxin in 400 µl H₂O und einem gleichen Volumen Freundschem kompletten Adjuvans wird einem Chinchilla-Kaninchen subcutan injiziert. Booster-Injektionen mit der gleichen Menge Antigen in Freundschem inkompletten Adjuvans erfolgen vier und zehn Wochen später. Zwölf Tage nach diesen Booster-Injektionen wird dem Tier Blut entnommen und Serum abgetrennt. IgGs werden über eine Säule aufgetrennt, die Protein A, gekoppelt an vernetzte Agarose enthält.

### Beispiel 4: Anti-idiotypische Antikörper

**Präparation anti-idiotypischer Antikörper** 40 mg Antikörper gegen das native aktivierte CryIA(b)-Toxin werden über eine Säule, die mit Aminoalkylagarose gefüllt ist, gegeben, an die 5 mg CryIA(b)-Toxin mit Hilfe von EDAC gekoppelt wurde. Die Säule wird mit 50 mM Tris-HCl pH 8 gespült, bis im Durchfluss kein Protein durch Absorption bei 280 nm mehr nachgewiesen werden kann. Die Elution gebundener Antikörper erfolgt mit einer Lösung von 10 mM Diethylamin und 0,5 % Desoxycholat pH 11,3. Die eluierten Fraktionen werden sofort durch die Zugabe von 1/50 Volumen 1 M Tris-HCl pH 8 neutralisiert. Etwa 3 mg IgG werden eluiert und nachfolgend über eine Säule gegeben, die mit Aminoalkylagarose gefüllt ist, an die CryIA(c) gekoppelt ist. Antikörper, die die in CryIA(b) und CryIA(c) identische Region erkennen, können binden, während Antikörper, die die variable Region von CryIA(b) (Geiser et al., 1986) erkennen, nicht an diese Säule binden können und deshalb im Durchfluss zu finden sind. Mit diesen Antikörpern (gegen CryIA(b)) (80 µg) wird ein Chinchilla-Kaninchen immunisiert. Booster-Injektionen werden in vierwöchigen Intervallen durchgeführt Zwölf Tage nach jeder Immunisierung wird dem Kaninchen Blut abgenommen und Serum präpariert. Die verschiedenen Seren werden auf ihre Fähigkeit hin getestet, an das 170 kDa *H. virescens* CryIA(b) BP in einem Western Blot zu binden. Die anti-idiotypischen IgGs werden gegen das CryIA(b) BP von *H. virescens* nach einem Verfahren von Madara et al. (1990) gereinigt.

**Bindung von anti-idiotypischen Antikörpern an BBM Proteine von Larven fünf verschiedener Insektenarten** Die oben erhaltenen anti-idiotypischen Antikörper werden verwendet, um zu untersuchen, ob die Toxin-Anheftungsstelle des Bindungsproteins bei den untersuchten Insekten konserviert ist. Sowohl das 170 kDa Bindungsprotein von *H. virescens* als auch das von *H. zea wird* spezifisch von den anti-idiotypischen Antikörpern erkannt. Die Antikörper erkennen nicht die BBM Bindungsproteine der *Spodoptera-Arten.*

Western Blots werden unter Verwendung einer PVDF-Membran gemäss Towbin et al. (1979) durchgeführt. Nach dem Transfer der BBMV Proteine auf die Membran und Blockierung der unspezifischen Bindungsstellen mit TBSTM werden die Membranen mit anti-idiotypischen Antikörpern (IgG-Fraktion 1:100 in TBSTM verdünnt) inkubiert. Die Membran-gebundenen Antikörper werden eine Stunde lang in mit TBSTM 1:5000 verdünnten, mit alkalischer Phosphatase markierten Ziegen-gegen-Kaninchen-Antikörpern inkubiert und anschliessend durch Umsetzung mit NBT und BCIP in 0,1 M NaHCO₃ und 1 mM MgCl₂ sichtbar gemacht.

### Beispiel 5: Verwendung von Antikörpern zur Isolierung von Toxinen

(a) Die parasporalen Kristalle von *B*. *thuringiensis kurstaki* HD1 werden wie in Beispiel 1 beschrieben gewonnen und proteolytisch aktiviert 0,5 mg der Toxin-Mischung wird über eine Säule gegeben, die gefüllt ist mit vernetzter Agarose, an die 5 mg Antikörper über Protein A gekoppelt wurden, die spezifisch mit CryIA(a) und CryIA(b) reagieren. Während CryIA(a) und CryIA(b) gebunden werden, befindet sich ein weiteres Toxin im Durchfluss und kann durch seine spezifische Reaktion mit den Toxin-Bindungsproteinen in *H. virescens* als CryIA(c) identifiziert werden.

Auf analoge Weise kann auch ein "neues" Toxin isoliert werden. Sind im Durchfluss mehrere "neue" Toxine vorhanden, erfolgen weitere Isolierungsschritte gemäss allgemein geläufiger Verfahren, beispielsweise mittels HPLC oder 2-D-Chromatographie, wie SDS-PAGE und Isoelektrofokussierung.

(b) Native BBMV werden mit Hilfe einer sogenannten Slot-Blot-Apparatur (Schleicher und Schüll) auf eine Membran transferiert, die danach in TBSTM abgesättigt wird (Beispiel 2). Anschliessend werden die Bindungsproteine, die mit CryIA(a) reagieren, mit diesem Toxin abgesättigt. Das in (a) erhaltene Toxin CryIA(c) wird mit ¹⁵I markiert und mit der beladenen Membran inkubiert. Die autoradiographische Messung zeigt, dass CryIA(c) an ein Bindungsprotein bindet, mit dem CryIA(a) nicht reagiert.

Dieses Verfahren kann auch zur Identifizierung eines "neuen" δ-Endotoxins angewendet werden.

### Referenzen

Bradford, M.M., Anal. Biochem. 72:248-254 (1976)
Caramori, T., Albertini, A.M., Galizzi, A., Gene 98:37-44 (1991)
Delafield, F.P., Somerville, H.J., Rittenberg, S.C., J. Bacteriol. 96:713-720 (1968)
Dixon, B., Biotechnology 9:415 (1991)
Ge, A.Z., Shivarova, N.I., Dean, D.H., Proc. Natl. Acad. Sci. USA 86:4037-4041 (1989)
Geiser, M., Schweitzer, S., Grimm, C., Gene 48:109-118 (1986)
Harlow, Lane, Antibodies - A Laboratory Manual, Cold Spring Harbor Laboratories, Cold
   Spring Harbor (1988)
Höfte, H., Whiteley, H.R., Microbiol. Rev. 53:242-255 (1989)
Huber-Lukac, M., Jaquet, F., Lüthy, P., Hütter, R., Braun, D.G., Infect. Immun,
   54:228-232 (1986)
Laemmli, U.K., Nature 227:680-685 (1970)
Madara, P.J., Banghart, L.R., Jack, L.J.W., Neira, L.M., Mather, I.H., Anal. Biochem.
   187:246-250 (1990)
van Rie, J., McGaughey, W.H., Johnson, D.E., Barnett, B.D., van Mellaert, H., Science
   247:72-74 (1990)
Sambrook, J., Fritsch, E.F., Maniatis, T., Molecular cloning, a laboratory manual, Cold
   Spring Harbor Laboratory, Cold Spring Harbor (1989)
Schnepf, H.E., Tomczak, K., Ortega, J.P., Whiteley, H.R., J. Biol. Chem.
   265:20923-20930 (1990)
Towbin, H. Staehelin, T., Gordon, J., Proc. Natl. Acad. Sci. U.S.A. 76:4350-4354 (1979)
Wolfersberger, M., Lüthy, P., Maurer, A., Parenti, P., Sacchi, F.V., Giordana, B., Hanozet,
   G.M., Comp. Biochem. Physiol. 86:301-308 (1987)
EP 292,435
EP 317,511
EP 342,633
EP 400,246

## Patentansprüche (Patentansprüche für folgende(n) Vertragsstaat(en): AT, BE, CH-LI, DE, DK, FR, GB, GR, IT, LU, MC, NL, PT, SE)

1. Ein Antikörper, der spezifisch mit den Bindungsproteinen *für Bacillus thuringiensis* δ-Endotoxine reagiert.

2. Der Antikörper gemäss Anspruch 1, dadurch gekennzeichnet, dass er spezifisch mit den Bindungsproteinen für das *Bacillus thuringiensis* CryIA(b) δ-Endotoxin reagiert.

3. Der Antikörper gemäss Anspruch 2, dadurch gekennzeichnet, dass er spezifisch mit dem Heliothis Bindungsprotein für das *Bacillus thuringiensis* CryIA(b) δ-Endotoxin reagiert.

4. Der Antikörper gemäss Anspruch 3, dadurch gekennzeichnet, dass er spezifisch mit dem H. virescens 170 kDa Bindungsprotein für das *Bacillus thuringiensis* CryIA(b) δ-Endotoxin reagiert

5. Der Antikörper gemäss Anspruch 1, dadurch gekennzeichnet, dass er erhältlich ist, indem Bindungsproteine für *Bacillus thuringiensis* δ-Endotoxine aus Bürstensaum (Brush Border)-Membranvesikeln des Mitteldarms von Insekten gewonnen und analog bekannter Verfahren zur Erzeugung des besagten Antikörpers eingesetzt werden.

6. Der Antikörper gemäss Anspruch 5, dadurch gekennzeichnet, dass es sich bei den δ-Endotoxinen um CryIA(b) δ-Endotoxine handelt.

7. Der Antikörper gemäss Anspruch 6, dadurch gekennzeichnet, dass es sich bei den Bindungsproteinen um Heliothis Bindungsproteine handelt.

8. Der Antikörper gemäss Anspruch 7, dadurch gekennzeichnet, dass es sich bei den Bindungsproteinen um H. virescens 170 kDa Bindungsproteine handelt.

9. Ein anti-idiotypischer Äntikörper, der spezifisch mit den Bindungsproteinen für *Bacillus* *thuringiensis* δ-Endotoxine reagiert.

10. Der anti-idiotypische Antikörper gemäss Anspruch 9, dadurch gekennzeichnet, dass der anti-idiotypische Antikörper spezifisch mit den Bindungsproteinen für das Bacillus thuringiensis CryIA(b) δ-Endotoxin reagiert.

11. Der anti-idiotypische Antikörper gemäss Anspruch 10, dadurch gekennzeichnet, dass der anti-idiotypische Antikörper spezifisch mit dem Heliothis Bindungsprotein für das *Bacillus thuringiensis* CryIA(b) δ-Endotoxin reagiert

12. Der anti-idiotypische Antikörper gemäss Anspruch 11, dadurch gekennzeichnet, dass der anti-idiotypische Antikörper spezifisch mit dem H. virescens 170 kDa Bindungsprotein für das *Bacillus thuringiensis* CryIA(b) δ-Endotoxin reagiert.

13. Der anti-idiotypische Antikörper gemäss Anspruch 9, dadurch gekennzeichnet, dass er erhältlich ist, indem Antikörper gegen das native aktivierte *Bacillus thudngiensis* δ-Endotoxin gewonnen und analog bekannter Verfahren zur Erzeugung des besagten Antikörpers eingesetzt werden.

14. Der anti-idiotypische Antikörper gemäss Anspruch 9, der spezifisch mit Bindungsproteinen für Bacillus thuringiensis δ-Endotoxine reagiert, dadurch gekennzeichnet, dass er erhältlich ist, indem (a) Antikörper gegen das native aktivierte *Bacillus thuringiensis* δ-Endotoxin gewonnen werden, (b) von diesen Antikörpern diejenigen Antikörper, die nicht gegen bestimmte Regionen des Toxins gerichtet sind, durch subtraktive Affinitätschromatographie abgetrennt werden, (c) mit diesen Antikörpern, die gegen die bestimmte Region des Toxins gerichtet sind erneut immunisiert wird, und (d) die Immunoglobuline selektioniert werden, die gegen die Bindungsproteine für *Bacillus thuringiensis* δ-Endotoxine gerichtet sind.

15. Der anti-idiotypische Antikörper gemäss Anspruch 13 oder 14, dadurch gekennzeichnet, dass es sich bei dem Toxin um CryIA(b) δ-Endotoxin handelt.

16. Der anti-idiotypische Antikörper gemäss Anspruch 15, dadurch gekennzeichnet, dass es sich bei den Bindungsproteinen um Heliothis Bindungsproteine handelt.

17. Der anti-idiotypische Antikörper gemäss Anspruch 16, dadurch gekennzeichnet, dass es sich bei den Bindungsproteinen um H. virescens 170 kDa Bindungsproteine handelt.

18. Ein Test-kit, der einen Antikörper enthält, der spezifisch mit den Bindungsproteinen für *Bacillus thuringiensis* δ-Endotoxine reagiert.

19. Ein Test-kit gemäss Anspruch 18, dadurch gekennzeichnet, dass der Antikörper spezifisch mit den Bindungsproteinen für das *Bacillus thuringiensis* CryIA(b) δ-Endotoxin reagiert.

20. Ein Test-kit gemäss Anspruch 19, dadurch gekennzeichnet, dass der Antikörper spezifisch mit dem Heliothis Bindungsprotein für das *Bacillus thuringiensis* CryIA(b) δ-Endotoxin reagiert.

21. Ein Test-kit gemäss Anspruch 20, dadurch gekennzeichnet, dass der Antikörper spezifisch mit dem H. virescens 170 kDa Bindungsprotein für das *Bacillus thuringiensis* CryIA(b) δ-Endotoxin reagiert.

22. Ein Verfahren zur Identifizierung eines *Bacillus thuringiensis* δ-Endotoxins, dadurch gekennzeichnet, dass (a) die Bindungsproteine für ein vorgegebenes δ-Endotoxin durch die Bindung von Antikörpern gemäss den Ansprüchen 1 bis 17 abgesättigt werden und (b) das Toxin an andere Bindungsproteine von vorbehandelten Mitteldarm-Membran-Proteinen bindet.

23. Ein Verfahren zur Bestimmung der Menge an vorhandenem Bindungsprotein mit Hilfe eines Antikörpers oder Test-kits gemäss einem der Ansprüche 1 bis 21.

24. Ein Verfahren zur Reduktion im Resistenzentwicklungspotential bei der Bekämpfung von Schädlingen, dadurch gekennzeichnet, dass Veränderungen in der Anzahl der Bindungsstellen bzw. der Bindungsproteine gemäss Anspruch 23 bestimmt werden und dementsprechend ein geeignetes Toxin oder Toxingemisch eingesetzt wird.

25. Verwendung eines Antikörpers oder Test-kits gemäss einem der Ansprüche 1 bis 21 in der Analyse der immunologischen Kreuzreaktivität von Toxin-Bindungsproteinen zur Bestimmung ihres Verwandschaftsgrads, dadurch gekennzeichnet, dass die Bindung eines Antikörpers gegen ein bestimmtes Bindungsprotein an immobilisierte Bindungsproteine verschiedener Herkunft bestimmt wird.

26. Verwendung eines Antikörpers gemäss einem der Ansprüche 1 bis 17 zur Analyse von Resistenzen, die auf das Fehlen des Bindungsproteins zurückzuführen sind.

27. Verwendung eines anti-idiotypischen Antikörpers gemäss einem der Ansprüche 9 bis 17 zur Analyse von Resistenzen, die auf das Fehlen der Bindungsstellen zurückzuführen sind.

28. Verwendung eines Antikörpers gemäss einem der Ansprüche 1 bis 17 zur Bestimmung der Verfügbarkeit und Zugänglichkeit eines Bindungsproteins für das entsprechende Toxin in verschiedenen Insekten.

29. Verwendung eines Test-kits gemäss einem der Ansprüche 18 bis 21 zur Bestimmung der Verfügbarkeit und Zugänglichkeit eines Bindungsproteins für das entsprechende Toxin in verschiedenen Insekten.

## Patentansprüche (Patentansprüche für folgende(n) Vertragsstaat(en): ES)

1. Ein Test-kit, der einen Antikörper enthält, der spezifisch mit den Bindungsproteinen für *Bacillus thuringiensis* δ-Endotoxine reagiert.

2. Ein Test-kit gemäss Anspruch 1, dadurch gekennzeichnet, dass der Antikörper spezifisch mit den Bindungsproteinen für das *Bacillus thuringiensis* CryIA(b) δ-Endotoxin reagiert.

3. Ein Test-kit gemäss Anspruch 2, dadurch gekennzeichnet, dass der Antikörper spezifisch mit dem Heliothis Bindungsprotein für das *Bacillus thuringiensis* CryIA(b) δ-Endotoxin reagiert.

4. Ein Test-kit gemäss Anspruch 3, dadurch gekennzeichnet, dass der Antikörper spezifisch mit dem H. virescens 170 kDa Bindungsprotein für das *Bacillus thuringiensis* CryIA(b) δ-Endotoxin reagiert.

5. Ein Verfahren zur Identifizierung eines *Bacillus thuringiensis* δ-Endotoxins, dadurch gekennzeichnet, dass (a) die Bindungsproteine für ein vorgegebenes δ-Endotoxin durch die Bindung von Antikörpern wie enthalten im Test-kit gemäss den ansprüchen 1 bis 4 äbgesättigt werden und (b) das Toxin an andere Bindungsproteine von vorbehandelten Mitteldarm-Membran-Proteinen bindet.

6. Ein Verfahren zur Bestimmung der Menge an vorhandenem Bindungsprotein mit Hilfe eines Antikörpers der spezifisch mit den Bindungsproteinen für *Bacillus thuringiensis* δ-Endotoxine reagiert, oder eines anti-idiotypischen Antikörpers, der spezifisch mit den Bindungsproteinen für *Bacillus thuringiensis* δ-Endotoxine reagiert, oder Test-kits gemäss einem der Ansprüche 1 bis 4.

7. Ein Verfahren zur Reduktion im Resistenzentwicklungspotential bei der Bekämpfung von Schädlingen, dadurch gekennzeichnet, dass Veränderungen in der Anzahl der Bindungsstellen bzw. der Bindungsproteine gemäss Anspruch 6 bestimmt werden und dementsprechend ein geeignetes Toxin oder Toxingemisch eingesetzt wird.

8. Verwendung eines Antikörpers, der spezifisch mit den Bindungsproteinen für *Bacillus* *thuringiensis* δ-Endotoxine reagiert, oder eines anti-idiotypischen Antikörpers, der spezifisch mit den Bindungsproteinen für *Bacillus thuringiensis* δ-Endotoxine reagiert, oder Test-kits gemäss einem der Ansprüche 1 bis 4 in der Analyse der immunologischen Kreuzreaktivität von Toxin-Bindungsproteinen zur Bestimmung ihres Verwandschaftsgrads, dadurch gekennzeichnet, dass die Bindung eines Antikörpers gegen ein bestimmtes Bindungsprotein an immobilisierte Bindungsproteine verschiedener Herkunft bestimmt wird.

9. Verwendung eines Antikörpers, der spezifisch mit den Bindungsproteinen für *Bacillus* *thuringiensis* δ-Endotoxine reagiert, oder eines anti-idiotypischen Antikörpers, der spezifisch mit den Bindungsproteinen für *Bacillus thuringiensis* δ-Endotoxine reagiert, zur Analyse von Resistenzen, die auf das Fehlen des Bindungsproteins zurückmrühren sind.

10. Verwendung eines Antikörpers, der spezifisch mit den Bindungsproteinen für *Bacillus* *thuringiensis* δ-Endotoxine reagiert, oder eines anti-idiotypischen Antikörpers, der spezifisch mit den Bindungsproteinen für *Bacillus thuringiensis* δ-Endotoxine reagiert, zur Analyse von Resistenzen, die auf das Fehlen der Bindungsstellen zurückzuführen sind.

11. Verwendung eines Antikörpers, der spezifisch mit den Bindungsproteinen für *Bacillus* *thuringiensis* δ-Endotoxine reagiert, oder eines anti-idiotypischen Antikörpers, der spezifisch mit den Bindungsproteinen für *Bacillus thuringiensis* δ-Endotoxine reagiert, zur Bestimmung der Verfügbarkeit und Zugänglichkeit eines Bindungsproteins für das entsprechende Toxin in verschiedenen Insekten.

12. Verwendung eines Test-kits gemäss einem der Ansprüche 1 bis 4 zur Bestimmung der Verfügbarkeit und Zugänglichkeit eines Bindungsproteins für das entsprechende Toxin in verschiedenen Insekten.

## Claims (Claims for the following Contracting State(s): AT, BE, CH/LI, DE, DK, FR, GB, GR, IT, LU, MC, NL, PT, SE.)

1. An antibody which reacts specifically with the binding proteins for *Bacillus* *thuringiensis* δ-endotoxins.

2. The antibody of claim 1, which reacts specifically with the binding proteins for the *Bacillus thuringiensis* CryIA(b) δ-endotoxin.

3. The antibody of claim 2, which reacts specifically with the *Heliothis* binding protein for the *Bacillus thuringiensis* CryIA(b) δ-endotoxin.

4. The antibody of claim 3, which reacts specifically with the *H*. *virescens* 170 kDA binding protein for the *Bacillus thuringiensis* CryIA(b) δ-endotoxin.

5. The antibody of claim 1, which is obtainable by producing binding proteins for *Bacillus* *thuringiensis* δ-endotoxins from brush border membrane vesicles of the midgut of insects and using those binding proteins analogously to known methods to produce the said antibody.

6. The antibody of claim 5, wherein the δ-endotoxins are CryIA(b) δ-endotoxins.

7. The antibody of claim 6, wherein the binding proteins are *Heliothis* binding proteins.

8. The antibody of claim 7, wherein the binding proteins are *H. virescens* 170 kDa binding proteins.

9. An anti-idiotype antibody which reacts specifically with the binding proteins for *Bacillus thuringiensis* δ-endotoxins.

10. The anti-idiotype antibody of claim 9, which reacts specifically with the binding proteins for the *Bacillus thuringiensis* CryIA(b) δ-endotoxin.

11. The anti-idiotype antibody of claim 10, which reacts specifically with the *Heliothis* binding protein for the *Bacillus thuringiensis* CryIA(b) δ-endotoxin.

12. The anti-idiotype antibody of claim 11, which reacts specifically with the *H. virescens* 170 kDa binding protein for the *Bacillus thuringiensis* CrylA(b) δ-endotoxin.

13. The anti-idiotype antibody of claim 9, which is obtainable by raising antibodies against the native activated *Bacillus thuringiensis* δ-endotoxin and using those antibodies analogously to known methods to produce the said antibody.

14. The anti-idiotype antibody of claim 9, which reacts specifically with binding proteins for *Bacillus thuringiensis* δ-endotoxins, which antibody is obtainable by (a) raising antibodies against the native activated *Bacillus thuringiensis* δ-endotoxin, (b) separating from said antibodies those antibodies which are not directed against specific regions of the toxin by subtractive affinity chromatography, (c) immunising repeatedly with those antibodies which are directed against the specific region of the toxin, and (d) selecting the immunoglobulins which are directed against the binding proteins for *Bacillus* *thuringiensis* δ-endotoxins.

15. The anti-idiotype antibody according to either claim 13 or claim 14, wherein the toxin is the CryIA(b) δ-endotoxin.

16. The anti-idiotype antibody according to claim 15, wherein the binding proteins are *Heliothis* binding proteins.

17. The anti-idiotype antibody according to claim 16, wherein the binding proteins are *H. virescens* 170 kDa binding proteins.

18. A test kit comprising an antibody which reacts specifically with the binding proteins for *Bacillus thuringiensis* δ-endotoxins.

19. A test kit according to claim 18, wherein the antibody reacts specifically with the binding proteins for the *Bacillus thuringiensis* CryIA(b) δ-endotoxin.

20. A test kit according to claim 19, wherein the antibody reacts specifically with the *Heliothis* binding protein for the *Bacillus thuringiensis* CryIA(b) δ-endotoxin.

21. A test kit according to claim 20, wherein the antibody reacts specifically with the *H. virescens* 170 kDa binding protein for the *Bacillus thuringiensis* CryIA(b) δ-endotoxin.

22. A method of identifying a *Bacillus thuringiensis* δ-endotoxin, which comprises (a) saturating the binding proteins for a given δ-endotoxin by binding antibodies according to claims 1 to 17, and (b) binding the toxin to other binding proteins of pretreated midgut-membrane proteins.

23. A method of determining the amount of binding protein present using an antibody or test kit as claimed in any one of claims 1 to 21.

24. A method of reducing the resistance development potential in pest control, which comprises determining the changes in the number of binding sites or binding proteins according to claim 23 and accordingly using a suitable toxin or mixture of toxins.

25. The use of an antibody or of a test kit as claimed in any one of claims 1 to 21 for analysing the immunological cross-reactivity of toxin-binding proteins to determine their degree of relationship by determining the binding of an antibody against a specific binding protein to immobilised binding proteins of different origin.

26. Use of an antibody as claimed in any one of claims 1 to 17 for analysing resistance which is attributable to the lack of the binding protein.

27. Use of an anti-idiotype antibody as claimed in any one of claims 9 to 17 for analysing resistance which is attributable to the lack of the binding sites.

28. Use of an antibody as claimed in any one of claims 1 to 17 for determining the availability and accessibility of a binding protein for the corresponding toxin in different insects.

29. Use of a test kit as claimed in any one of claims 18 to 21 for determining the availability and accessibility of a binding protein for the corresponding toxin in different insects.

## Claims (Claims for the following Contracting State(s): ES)

1. A test kit comprising an antibody which reacts specifically with the binding proteins for *Bacillus thuringiensis* δ-endotoxins.

2. A test kit according to claim 1, wherein the antibody reacts specifically with the binding proteins for the *Bacillus thuringiensis* CryIA(b) δ-endotoxin.

3. A test kit according to claim 2, wherein the antibody reacts specifically with the *Heliothis* binding protein for the *Bacillus thuringiensis* CryIA(b) δ-endotoxin.

4. A test kit according to claim 3, wherein the antibody reacts specifically with the *H. virescens* 170 kDa binding protein for the *Bacillus thuringiensis* CryIA(b) δ-endotoxin.

5. A method of identifying a *Bacillus thuringiensis* δ-endotoxin, which comprises (a) saturating the binding proteins for a given δ-endotoxin by binding antibodies as contained in the test kit according to claims 1 to 4, and (b) binding the toxin to other binding proteins of pretreated midgut-membrane proteins.

6. A method of determining the amount of binding protein present using an antibody which reacts specifically with the binding proteins for *Bacillus thuringiensis* δ-endotoxins, or an anti-idiotype antibody which reacts specifically with the binding proteins for *Bacillus thuringiensis* δ-endotoxins, or a test kit according to any one of claims 1 to 4.

7. A method of reducing the resistance development potential in pest control, which comprises determining the changes in the number of binding sites or binding proteins according to claim 6 and accordingly using a suitable toxin or mixture of toxins.

8. The use of an antibody which reacts specifically with the binding proteins for *Bacillus* *thuringiensis* δ-endotoxins, or of an anti-idiotype antibody which reacts specifically with the binding proteins for *Bacillus thuringiensis* δ-endotoxins, or of a test kit as claimed in any one of claims 1 to 4 for analysing the immunological cross-reactivity of toxin-binding proteins to determine their degree of relationship by determining the binding of an antibody against a specific binding protein to immobilised binding proteins of different origin.

9. Use of an antibody which reacts specifically with the binding proteins for *Bacillus* *thuringiensis* δ-endotoxins or of an anti-idiotype antibody which reacts specifically with the binding proteins for *Bacillus thuringiensis* δ-endotoxins for analysing resistance which is attributable to the lack of the binding protein.

10. Use of an antibody which reacts specifically with the binding proteins for *Bacillus* *thuringiensis* δ-endotoxins or of an anti-idiotype antibody which reacts specifically with the binding proteins for *Bacillus thuringiensis* δ-endotoxins for analysing resistance which is attributable to the lack of the binding sites.

11. Use of an antibody which reacts specifically with the binding proteins for *Bacillus* *thuringiensis* δ-endotoxins or of an anti-idiotype antibody which reacts specifically with the binding proteins for *Bacillus thuringiensis* δ-endotoxins for determining the availability and accessibility of a binding protein for the corresponding toxin in different insects.

12. Use of a test kit as claimed in any one of claims 1 to 4 for determining the availability and accessibility of a binding protein for the corresponding toxin in different insects.

## Revendications (Revendications pour l'(les) Etat(s) contractant(s) suivant(s): AT, BE, CH/LI, DE, DK, FR, GB, GR, IT, LU, MC, NL, PT, SE)

1. Anticorps qui réagit spécifiquement avec les protéines de liaison pour des δ-endotoxines de *Bacillus* *thuringiensis.*

2. Anticorps selon la revendication 1, caractérisé en ce qu'il réagit spécifiquement avec les protéines de liaison pour la δ-endotoxine CryIA(b) de *Bacillus* *thuringiensis.*

3. Anticorps selon la revendication 2, caractérisé en ce qu'il réagit spécifiquement avec la protéine de liaison d*'Heliothis* pour la δ-endotoxine CryIA(b) de *Bacillus* *thuringiensis.*

4. Anticorps selon la revendication 3, caractérisé en ce qu'il réagit spécifiquement avec la protéine de liaison de 170 kDa de *H. virescens* pour la δ-endotoxine CryIA(b) de *Bacillus thuringiensis.*

5. Anticorps selon la revendication 1, caractérisé en ce qu'on peut l'obtenir en recueillant des protéines de liaison pour des δ-endotoxines de *Bacillus thuringiensis* à partir de vésicules de membranes de bordure en brosse de l'intestin moyen d'insectes, et en les utilisant, selon des procédés connus, pour la production dudit anticorps.

6. Anticorps selon la revendication 5, caractérisé en ce que les 6-endotoxines sont des δ-endotoxines CryIA(b).

7. Anticorps selon la revendication 6, caractérisé en ce que les protéines de liaison sont des protéines de liaison d*'Heliothis.*

8. Anticorps selon la revendication 7, caractérisé en ce que les protéines de liaison sont des protéines de liaison de 170 kDa de *H. virescens.*

9. Anticorps anti-idiotypique réagissant spécifiquement avec les protéines de liaison pour des δ-endotoxines de *Bacillus thuringiensis.*

10. Anticorps anti-idiotypique selon la revendication 9, caractérisé en ce que l'anticorps anti-idiotypique réagit spécifiquement avec les protéines de liaison pour la δ-endotoxine CryIA(b) de *Bacillus thuringiensis.*

11. Anticorps anti-idiotypique selon la revendication 10, caractérisé en ce que l'anticorps anti-idiotypique réagit spécifiquement avec la protéine de liaison d*'Heliothis* pour la 6-endotoxine CryIA(b) de *Bacillus* *thuringiensis.*

12. Anticorps anti-idiotypique selon la revendication 11, caractérisé en ce que l'anticorps anti-idiotypique réagit spécifiquement avec la protéine de liaison de 170 kDa de *H*. *virescens* pour la δ-endotoxine CryIA(b) de *Bacillus* *thuringiensis.*

13. Anticorps anti-idiotypique selon la revendication 9, caractérisé en ce qu'on peut l'obtenir en recueillant des anticorps dirigés contre la δ-endotoxine native activée de *Bacillus thuringiensis* et en utilisant des procédés connus analogues pour la production dudit anticorps.

14. Anticorps anti-idiotypique selon la revendication 9, réagissant spécifiquement avec des protéines de liaison pour des δ-endotoxines de *Bacillus thuringiensis,* caractérisé en ce qu'on peut l'obtenir (a) en recueillant des anticorps dirigés contre la δ-endotoxine native activée de *Bacillus thuringiensis,* (b) en séparant d'avec ces anticorps, par chromatographie d'affinité soustractive, les anticorps qui ne sont pas dirigés contre des régions déterminées de la toxine, (c) en effectuant une nouvelle immunisation avec ces anticorps qui sont dirigés contre la région déterminée de la toxine, et (d) en sélectionnant les immunoglobulines qui sont dirigées contre les protéines de liaison pour des δ-endotoxines de *Bacillus thuringiensis.*

15. Anticorps anti-idiotypique son la revendication 13 ou 14, caractérisé en ce que la toxine est la δ-endotoxine CryIA(b).

16. Anticorps anti-idiotypique selon la revendication 15, caractérisé en ce que les protéines de liaison sont des protéines de liaison d*'Heliothis.*

17. Anticorps anti-idiotypique selon la revendication 16, caractérisé en ce que les protéines de liaison sont des protéines de liaison de 170 kDa de H. *virescens.*

18. Nécessaire d'essai, contenant un anticorps qui réagit spécifiquement avec les protéines de liaison pour des 6-endotoxines de *Bacillus thuringiensis.*

19. Nécessaire d'essai selon la revendication 18, caractérisé en ce que l'anticorps réagit spécifiquement avec les protéines de liaison pour la 6-endotoxine CryIA(b) de *Bacillus thuringiensis.*

20. Nécessaire d'essai selon la revendication 19, caractérisé en ce que l'anticorps réagit spécifiquement avec la protéine de liaison d*'Heliothis* pour la δ-endotoxine CryIA(b) de *Bacillus thuringiensis.*

21. Nécessaire d'essai selon la revendication 20, caractérisé en ce que l'anticorps réagit spécifiquement avec la protéine de liaison de 170 kDa de *H. virescens* pour la δ-endotoxine CryIA(b) de *Bacillus thuringiensis.*

22. Procédé pour l'identification d'une δ-endotoxine de *Bacillus thuringiensis,* caractérisé en ce que (a) on sature les protéines de liaison pour une δ-endotoxine préétablie, par la fixation d'anticorps selon les revendications 1 à 17, et (b) la toxine est liée à d'autres protéines de liaison de protéines prétraitées de membrane d'intestin moyen.

23. Procédé pour la détermination de la quantité de protéine de liaison présente, à l'aide d'un anticorps ou d'un nécessaire d'essai selon l'une des revendications 1 à 21.

24. Procédé pour la réduction du potentiel de développement de résistance dans la lutte contre des ravageurs, caractérisé en ce que l'on détermine selon la revendication 23 des modifications du nombre des sites de liaison ou des protéines de liaison et on utilise de façon correspondante une toxine appropriée ou un mélange de toxines appropriées.

25. Utilisation d'un anticorps ou d'un nécessaire d'essai selon l'une des revendications 1 à 21, dans l'analyse de la réactivité croisée immunologique de protéines de liaison-toxines pour la détermination de leur degré d'affinité, caractérisée en ce que l'on détermine la liaison d'un anticorps, dirigé contre une protéine de liaison déterminée, à des protéines de liaison immobilisées d'origine variée.

26. Utilisation d'un anticorps selon l'une des revendications 1 à 17, pour l'analyse de résitances qui doivent être attribuées à l'absence de la protéine de liaison.

27. Utilisation d'un anticorps anti-idiotypique selon l'une des revendications 9 à 17, pour l'analyse de résistances qui doivent être attribuées à l'absence des sites de liaison.

28. Utilisation d'un anticorps selon l'une des revendications 1 à 17, pour la détermination de la disponibilité et de l'accessibilité d'une protéine de liaison pour la toxine correspondante chez divers insectes.

29. Utilisation d'un nécessaire d'essai selon l'une des revendications 18 à 21, pour la détermination de la disponibilité et de l'accessibilité d'une protéine de liaison pour la toxine correspondante chez divers insectes.

## Revendications (Revendications pour l'(les) Etat(s) contractant(s) suivant(s): ES)

1. Nécessaire d'essai, contenant un anticorps qui réagit spécifiquement avec les protéines de liaison pour des δ-endotoxines de *Bacillus thuringiensis.*

2. Nécessaire d'essai selon la revendication 1, caractérisé en ce que l'anticorps réagit spécifiquement avec les protéines de liaison pour la δ-endotoxine CryIA(b) de *Bacillus thuringiensis.*

3. Nécessaire d'essai selon la revendication 2, caractérisé en ce que l'anticorps réagit spécifiquement avec la protéine de liaison *d'Heliothis* pour la δ-endotoxine CryIA(b) de *Bacillus thuringiensis.*

4. Nécessaire d'essai selon la revendication 3, caractérisé en ce que l'anticorps réagit spécifiquement avec la protéine de liaison de 170 kDa de *H*. *virescens* pour la δ-endotoxine CryIA(b) de *Bacillus thuringiensis.*

5. Procédé pour l'identification d'une δ-endotoxine de *Bacillus thuringiensis,* caractérisé en ce que (a) on sature les protéines de liaison pour une δ-endotoxine préétablie, par la fixation d'anticorps tels que contenus dans le nécessaire selon les revendications 1 à 4 et (b) la toxine est liée à d'autres protéines de liaison de protéines prétraitées de membrane d'intestin moyen.

6. Procédé pour la détermination de la quantité de protéine de liaison présente à l'aide d'un anticorps qui réagit spécifiquement avec les protéines de liaison pour des 6-endotoxines de *Bacillus thuringiensis,* ou d'un anticorps anti-idiotypique qui réagit spécifiquement avec les protéines de liaison pour des δ-endotoxines de *Bacillus* *thuringiensis,* ou du nécessaire d'essai selon l'une des revendications 1 à 4.

7. Procédé pour la réduction du potentiel de développement de résistance dans la lutte contre des ravageurs, caractérisé en ce que l'on détermine selon la revendication 6 des modifications du nombre des sites de liaison ou des protéines de liaison et on utilise de façon correspondante une toxine appropriée ou un mélange de toxines appropriées.

8. Utilisation d'un anticorps qui réagit spécifiquement avec les protéines de liaison pour des δ-endotoxines de *Bacillus thuringiensis,* ou d'un anticorps anti-idiotypique qui réagit spécifiquement avec les protéines de liaison pour des δ-endotoxines de *Bacillus thuringiensis,* ou d'un nécessaire d'essai selon l'une des revendications 1 à 4, dans l'analyse de la réactivité croisée immunologique de protéines de liaison-toxine pour la détermination de leur degré d'affinité, caractérisé en ce que l'on détermine la liaison d'un anticorps, dirigé contre une protéine de liaison déterminée, à des protéines de liaison immobilisées d'origine variée.

9. Utilisation d'un anticorps qui réagit spécifiquement avec les protéines de liaison pour des δ-endotoxines de *Bacillus thuringiensis,* ou d'un anticorps anti-idiotypique qui réagit spécifiquement avec les protéines de liaison pour des δ-endotoxines de *Bacillus thuringiensis,* pour l'analyse de résistances qui doivent être attribuées à l'absence de la protéine de liaison.

10. Utilisation d'un anticorps qui réagit spécifiquement avec les protéines de liaison pour des δ-endotoxines de *Bacillus thuringiensis, ou* d'un anticorps anti-idiotypique qui réagit spécifiquement avec les protéines de liaison pour des δ-endotoxines de *Bacillus thuringiensis,* pour l'analyse de résistances qui doivent être attribuées à l'absence des sites de liaison.

11. Utilisation d'un anticorps qui réagit spécifiquement avec les protéines de liaison pour des δ-endotoxines de *Bacillus thuringiensis,* ou d'un anticorps anti-idiotypique qui réagit spécifiquement avec les protéines de liaison pour des δ-endotoxines de *Bacillus thuringiensis,* pour la détermination de la disponibilité et de l'accessibilité d'une protéine de liaison pour la toxine correspondante chez divers insectes.

12. Utilisation d'un nécessaire d'essai selon l'une des revendications 1 à 4, pour la détermination de la disponibilité et de l'accessibilité d'une protéine de liaison pour la toxine correspondante chez divers insectes.
